**Europäisches Patentamt**

**European Patent Office**

**Office européen des brevets**

(19)

(11) Veröffentlichungsnummer : **0 212 121 B1**

(12) # EUROPÄISCHE PATENTSCHRIFT

(45) Veröffentlichungstag der Patentschrift :
06.02.91 Patentblatt 91/06

(51) Int. Cl.$^5$ : **C07D 307/60**

(21) Anmeldenummer : **86108179.2**

(22) Anmeldetag : **14.06.86**

(54) Verfahren zur kontinuierlichen Abscheidung von Maleinsäureanhydrid aus gasförmigen Reaktionsgemischen.

(30) Priorität : **19.06.85 DE 3521768**

(43) Veröffentlichungstag der Anmeldung :
**04.03.87 Patentblatt 87/10**

(45) Bekanntmachung des Hinweises auf die
Patenterteilung :
**06.02.91 Patentblatt 91/06**

(84) Benannte Vertragsstaaten :
**BE DE FR GB IT NL**

(56) Entgegenhaltungen :
**EP-A- 0 149 144**

(73) Patentinhaber : **BASF Aktiengesellschaft
Carl-Bosch-Strasse 38
D-6700 Ludwigshafen (DE)**

(72) Erfinder : **Malsch, Klaus-Dieter, Dr.
Amselweg 14
D-6707 Schifferstadt (DE)**
Erfinder : **Fischer, Rolf, Dr.
Bergstrasse 98
D-6900 Heidelberg (DE)**
Erfinder : **Schnabel, Rolf, Dr.
Frankenstrasse 22
D-6707 Schifferstadt (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein Verfahren zur kontinuierlichen Abscheidung von Maleinsäureanhydrid (MSA) aus gasförmigen Reaktionsgemischen durch Alkohole und Dicarbonsäurediester.

Aus der DE-OS 25 43 673 ist bekannt, daß man 1,4-Butandiol aus MSA dadurch herstellen kann, daß man in einer ersten Stufe MSA mit einem einwertigen aliphatischen Alkohol mit 1 bis 6 Kohlenstoffatomen verestert und den Maleinsäuredialkylester in einer zweiten Stufe katalytisch hydriert. Nach einem in der DE-PS 28 45 905 beschriebenen Verfahren führt man die 1,4-Butandiol-Bildung in einer Stufe durch, wobei man ein Gemisch aus MSA und dem Alkohol unter Verzicht auf eine vollständige Veresterung des MSA unmittelbar katalytisch hydriert.

In der DE-OS 31 06 819 wird der Vorschlag gemacht, das MSA aus einem gasförmigen Reaktionsgemisch durch eine Wäsche mit 1,4-Butandiol abzuscheiden. Das dabei erhältliche Gemisch wird zum 1,4-Butandiol hydriert. Man muß hierbei zwar kein Lösungsmittel abtrennen, doch gelingt es nicht, sowohl hohe Konzentrationen an MSA bzw. an monomeren MSA-Folgeprodukten im Absorbat zu erhalten als auch die unerwünschte Bildung von Oligomeren und Polymeren aus Butandiol und MSA zu verhindern. Beides ist aber wünschenswert, denn hohe Raum-Zeit-Ausbeuten kann man nur mit hohen Absorbat-Konzentrationen erzielen, und Oligomere und Polymere beeinträchtigen die Katalysatoren bei der nachfolgenden Hydrierung.

Versucht man, für eine Butandiol-Herstellung geeignete Gemische aus Alkanol und MSA herzustellen, so ergeben sich durch den hohen Gehalt des Abgases an Alkanol so erhebliche technische Schwierigkeiten, daß eine Alkoholwäsche mit wirtschaftlich vertretbarem Aufwand nicht betrieben werden kann. Es bestand deshalb die Aufgabe, ein Verfahren zum Auswaschen von MSA aus Reaktionsgasen mit Lösungsmitteln zu finden, mit dem man auf möglichst wirtschaftliche Weise ein flüssiges Gemisch erhält, das sich vorteilhaft zur Gewinnung von 1,4-Butandiol hydrieren läßt.

Bei dem Verfahren der Erfindung, durch das diese Aufgabe gelöst wird, nimmt man die kontinuierliche Abscheidung von MSA aus gasförmigen Reaktionsgemischen, die man bei der katalytischen Oxidation von Kohlenwasserstoffen erhält, durch Behandlung des gasförmigen Reaktionsgemischs mit Lösungsmitteln so vor, daß man das MSA enthaltende gasförmige Reaktionsgemisch mit einem einwertigen aliphatischen Alkohol mit 1 bis 3 oder 5 bis 8 Kohlenstoffatomen in Kontakt bringt, die dabei anfallenden gasförmigen Stoffe im Gegenstrom mit einem Dicarbonsäurediester in Berührung bringt und das flüssige Verfahrensprodukt dem Sumpf entnimmt.

Nach dem erfindungsgemäßen Verfahren bringt man ein MSA enthaltendes gasförmiges Reaktionsgemisch zum Zwecke der MSA-Absorption mit dem Alkohol in Kontakt. Die Reaktionsgaszufuhr erfolgt bei einer zweckmäßigen Ausführungsform des Verfahrens unterhalb der Oberfläche des flüssigen Alkohols, z.B. durch ein Tauchrohr. Das Reaktionsgemisch kann aber auch direkt von unten in eine Absorptionskolonne eingeleitet werden, wo ihm flüssiger Alkohol entgegenströmt. Die Arbeitsweise, bei der man die MSA-Absorption in einer Kolonne oder in mehreren hintereinandergeschalteten Kolonnen durchführt, ist bevorzugt. Man kann das Reaktionsgemisch auch mit gasförmigem Alkohol in Kontakt bringen.

MSA enthaltende Reaktionsgemische werden z.B. nach der an sich bekannten katalytischen Oxidation von Kohlenwasserstoffen wie Butene, Butan oder auch Benzol, erhalten. Bei diesem bekannten Oxidationsverfahren strömt aus dem Reaktor ein gasförmiges Reaktionsgemisch mit einer Temperatur von 250 bis 600°C, das je Nm$^3$ bis 40 g Maleinsäureanhydrid enthält. Außerdem enthalten die gasförmigen Reaktionsgemische neben nicht umgesetztem Kohlenwasserstoff Wasser, Kohlenmonoxid und Kohlendioxid.

Als Kolonnen sind z.B. Absorptionskolonnen, wie Glockenboden-, Füllkörper- und Siebbodenkolonnen mit Bodenzahlen von 1 bis 50, vorzugsweise 5 bis 20 geeignet. Man arbeitet zweckmäßigerweise so, daß man das das MSA enthaltende gasförmige Reaktionsgemisch in den Kolonnensumpf, den Alkohol in den unteren Teil der Kolonne, vorteilhaft oberhalb des Kolonnensumpfes und den Dicarbonsäurediester über den Kopf in die Kolonne einleitet. Die Temperaturen liegen im Sumpf bei 0 bis 280°C, in der Kolonne bei -20 bis 150°C und am Kopf der Kolonne bei -20 bis 130°C.

Als einwertige aliphatische Alkohole mit 1 bis 3 oder 5 bis 8 C-Atomen kommen die entsprechenden Alkanole, wie Methanol, Ethanol, Propanol, Isopropanol, n-Amylalkohol, iso-Amylalkohol, n-Hexanol, 2-Ethylhexanol in Frage. Als Dicarbonsäurediester verwendet man bevorzugt Diester von C$_4$-Dicarbonsäuren, wie die Diester, die einerseits aus der Bernstein-, Fumar- oder Maleinsäure und andererseits aus dem eingesetzten Alkohol bestehen. Bezogen auf 1 Mol MSA im gasförmigen Ausgangsgemisch wendet man 0,2 bis 10 Mol Butanol und 0,2 bis 20 Mol Maleinsäuredibutylester an.

Nach einer bevorzugten Arbeitsweise, bei der man die kontinuierliche Abscheidung des MSA in einer Kolonne vornimmt, werden das Gasgemisch und der Alkohol wie geschildert in den Sumpf bzw. den unteren Teil der Kolonne eingeleitet. Als unterer Teil der Kolonne wird der Teil oberhalb des Sumpfes bis zum zweiten bis fünften Boden verstanden. Oberhalb der Einleitung des Alkohols, also oberhalb des zweiten bis fünften Bodens, wird eine eventuell auftretende zweite flüssige Phase, die hauptsächlich aus Wasser besteht, aus der Kolonne ausgeschleust. Aus dem mittleren Teil der Kolonne, unter dem der Bereich bis zu fünf Böden oberhalb der Entnahmestellen für die wäßrige Phase verstanden wird, schleust man ein flüssiges Gemisch aus, aus dem man in einer separaten Destillationsvorrichtung, wie einer Destillationskolonne, die leichter als der Dicarbonsäurediester siedenden Anteile abdestilliert, die man in den unteren Teil der Kolonne zurückführt. Der bei dieser separaten Destillation als Sumpfprodukt anfallende Dicarbonsäurediester wird der Absorptionskolonne über Kopf zugeführt. Die Kolonne wird dabei so betrieben, daß die Temperatur im untersten Boden zwischen 0 und 150°C, vorzugsweise zwischen 25 und 100°C liegt, sich im mittleren Teil der Kolonne eine Temperatur von -20 bis 150°C, vorzugsweise von 0 bis 65°C und am Kolonnenkopf eine Temperatur von -20 bis 130°C, vorzugsweise 0 bis 40°C einstellt.

Unter den Bedingungen der erfindungsgemäßen Absorption wird das MSA in der Kolonne überwiegend in den Maleinsäuremono- und -dialkylester überführt. Die gasförmigen Begleitstoffe des MSA werden über Kopf der Kolonne abgeleitet, während eventuell ein Teil des Wassers die Kolonne durch den Seitenabzug verläßt. Das flüssige Verfahrensprodukt, das man dem Sumpf der Kolonne entnimmt, hat z.B. folgende Zusammensetzung : 0,01 bis 93 Gew.% Alkohol, 0,7 bis 79 Gew.% Maleinsäuremonoalkylester, 0,5 bis 95 Gew.% Maleinsäuredialkylester, bis zu 50 Gew.% MSA, Maleinsäure und Fumarsäure. Der Maleinsäuredialkylester enthält gewöhnlich erhebliche Anteile an dem isomeren Fumarsäuredialkylester. Das flüssige Gemisch eignet sich hervorragend für die Hydrierung zur Gewinnung von 1,4-Butandiol. Hierzu empfiehlt es sich, das flüssige Gemisch zum Zwecke der Vervollständigung der Veresterung z.B. auf 100 bis 250°C zu erhitzen, wobei man das Reaktionswasser mit dem überschüssigen Alkohol abtreibt und somit den für das erfindungsgemäße Verfahren benötigten Alkohol zurückgewinnt. Der Maleinsäuredialkylester kann dann auf an sich bekannte Weise zu 1,4-Butandiol hydriert werden.

Das neue Verfahren ermöglicht es, das MSA aus den Reaktionsgasen quantitativ und unter weitgehender Vermeidung von Absorptionsmittel-Verlusten in ein flüssiges Gemisch zu überführen, das eine hohe Konzentration an dem Halb- und Diester der Maleinsäure aufweist und das sich hervorragend für die Hydrierung zur Gewinnung von 1,4-Butandiol eignet. Überraschenderweise treten bei der erfindungsgemäßen Arbeitsweise störende Abscheidungen von Kristallen aus Maleinsäure und/oder Fumarsäure nicht auf.

## Beispiel 1

(s. Figur)

Ein Stickstoffstrom von 200 Nl/h (Normalliter pro Stunde) wird in einem Sättigungsgefäß mit MSA und mit Waserdampf so beladen, daß er 0,5 Vol.% (4,5 g/h) MSA und 5 Vol.% Waser enthält. Der so hergestellte Gasstrom entspricht bezüglich dieser Komponenten weitgehend einem gasförmigen Reaktionsgemisch, das man bei der bekannten Luftoxidation von Butan, Butenen oder auch Benzol zum Zwecke der Herstellung von MSA erhält.

Das Gasgemisch (1) wird in einem zu Versuchsbeginn mit 1/2 Liter n-Amylalkohol beschickten Kolben (2) bei einer Temperatur von 60°C getaucht eingeleitet. Der Kolben ist mit einer Zuleitung (3) ausgerüstet. Durch eine Ableitung (4) wird in regelmäßigen Zeitabständen flüssiges Reaktionsgemisch entnommen, so daß der Flüssigkeitsstand im Kolben konstant bleibt. Auf dem Kolben ist ein Schuß einer Glockenbodenkolonne (5) mit drei Glockenböden angebracht, der mit einem Probeentnahmestutzen (6) ausgerüstet ist. Die Temperatur hält man in diesem Teil der Kolonne auf 50°C. Über die Zuleitung (3) werden 10 ml/h Amylalkohol in den Kolben (2) eingeleitet. Auf dem Kolonnenteil (5) befindet sich ein mit einem Ablaßstutzen (8) versehener auf 25°C gehaltener Phasenabscheider (7), durch den man eine möglicherweise auftretende zweite flüssige Phase mit höherer Dichte (wäßrige Phase) aus der Kolonne ausschleusen kann. Auf dem Phasenabscheider (7) sind weitere Schüsse mit insgesamt 18 Glockenböden (9) angebracht, die auf 25°C gehalten werden.

Drei Böden über dem Phasenabscheider (7) ist ein Ablaßstutzen (10) installiert, mit dem man das flüssige Absorbat aus dem darüberliegenden Kolonnenteil ausschleust und einer Destillationsvorrichtung zuführt. Über den Kopf der Kolonne werden 20 ml/h Bernsteinsäurediamylester zugeführt (11). Gleichzeitig verläßt ein Gasstrom den Kolonnenkopf (12).

Nach fünftägiger Versuchsdauer stellt sich ein stationärer Zustand ein, und man erhält die folgenden Ergebnisse :

Das gasförmig zugeführte MSA (4,5 g/h) findet sich wieder im flüssigen Gemisch, das sich im unteren Teil der Apparatur ansammelt, und zwar überwiegend in Form der Ester. Durch GC- und HPLC-Analyse des Kolbenaustrags (4) wird folgende Zusammensetzung ermittelt : 13,5 Gew.% n-Amylalkohol, 61 Gew.% Maleinsäuremonoamylester, 18 Gew.% Maleinsäurediamylester und 7 Gew.% MSA, Maleinsäure und Fumarsäure. Bereits am Ablaßstutzen (6) lassen sich weder MSA, Maleinsäure, Fumarsäure noch Halbester nachweisen. Am Phasenabscheider (7) werden 6 bis 9 g/h einer wäßrigen Phase ausgeschleust, die 6 bis 7 Gew.% n-Amylalkohol enthält. Das über den Ablaßstutzen (10) entnommene flüssige Absorbat enthält 6,1 Gew.% n-Amylalkohol und 94 Gew.% Bernsteinsäurediamylester. Die Amylalkohol-Konzentration geht im oberen Teil der Kolonne (9) soweit zurück, daß mit dem Abgas (12) neben 3,0 g/h Wasser und weniger als 0,01 g/h Bernsteinsäurediamylester weniger als 0,01 g/h n-Amylalkohol ausgetragen werden.

Beispiel 2

(s. Figur)

Man verfährt wie in Beispiel 1 beschrieben, wobei man jedoch anstelle von n-Amylalkohol Methanol als Absorptionsmittel für MSA und anstelle von Bernsteinsäurediamylester Maleinsäuredimethylester für die Abgaswäsche verwendet. Wie in Beispiel 1 wird der Kolben (2) bei Versuchsbeginn mit 500 ml des Alkohols beschickt ; über die Zuleitung (3) werden 10 ml/h Methanol zugeführt, und auf den Kopf der Kolonne werden 20 ml/h Maleinsäuredimethylester gegeben (11).

Der Teil der Kolonne oberhalb des Phasenabscheiders (7) wird auf 20°C gehalten. Nach fünftägiger Versuchsdauer stellt sich ein stationärer Zustand ein, und man erhält die folgenden Ergebnisse :
Das gasförmige MSA (4,5 g/h) findet sich wieder im flüssigen Gemisch, das sich im unteren Teil der Apparatur ansammelt, und zwar überwiegend in Form der Ester. Durch GC- und HPLC-Analyse des Kolbenaustrags (4) wird folgende Zusammensetzung ermittelt: 5,9 Gew.% Methanol, 69 Gew.% Maleinsäuremonomethylester ; 1,1 Gew.% Maleinsäuredimethylester und 22 Gew.% MSA.

Bereits am Ablaßstutzen (6) lassen sich weder MSA, Maleinsäure, Fumarsäure noch Halbester nachweisen. Am Phasenabscheider (7) fällt keine wäßrige Phase an.

Beispiel 3

(s. Figur)

Man verfährt wie in Beispiel 1 beschrieben, wobei man jedoch anstelle von n-Amylalkohol Ethanol als Absorptionsmittel für MSA und anstelle von Bernsteinsäurediamylester Fumarsäurediethylester für die Abgaswäsche verwendet. Wie in Beispiel 1 wird der Kolben (2) bei Versuchsbeginn mit 500 ml des Alkohols beschickt ; über die Zuleitung (3) werden 10 ml/h Ethanol zugeführt, und auf den Kopf der Kolonne werden 20 ml/h Fumarsäurediethylester gegeben (11).

Der Teil der Kolonne oberhalb des Phasenabscheiders (7) wird auf 20°C gehalten. Nach fünftägiger Versuchsdauer stellt sich ein stationärer Zustand ein, und man erhält die folgenden Ergebnisse :
Das gasförmige MSA (4,5 g/h) findet sich wieder im flüssigen Gemisch, das sich im unteren Teil der Apparatur ansammelt, und zwar überwiegend in Form der Ester. Durch GC- und HPLC-Analyse des Kolbenaustrags (4) wird folgende Zusammensetzung ermittelt: 2,5 Gew.% Ethanol, 79 Gew.% Maleinsäuremonoethylester ; 17,1 Gew.% Maleinsäurediethylester und <0,1 Gew.% MSA.

Bereits am Ablaßstutzen (6) lassen sich weder MSA, Maleinsäure, Fumarsäure noch Halbester nachweisen. Am Phasenabscheider (7) fällt keine wäßrige Phase an.

Beispiel 4

(s. Figur, Vergleichsversuch)

Man verfährt wie in Beispiel 1 bis 3 beschrieben, wobei man jedoch auf die Zufuhr des Alkohols (3) verzichtet und den am Kopf der Kolonne zugeführten Diester (11 nicht über den Ablaßstutzen (10) am oberen Teil der Kolonne, sondern über die Ableitung (4) am Kolben (2) wieder entnimmt.

Innerhalb eines Tages kristallisiert Maleinsäure in der Kolonne aus, so daß die Absorption abgebrochen werden muß. Erhöht man in dem Versuch die Zugabe des Diesters auf 100 ml/h, so läßt sich die störende Kristallbildung zwar verhindern, aber man erhält dabei ein flüssiges Verfahrensprodukt, das vor einer Hydrierung zum Zwecke der Herstellung von 1,4-Butandiol eine zusätzliche Anreicherungsstufe erforderlich machen würde.

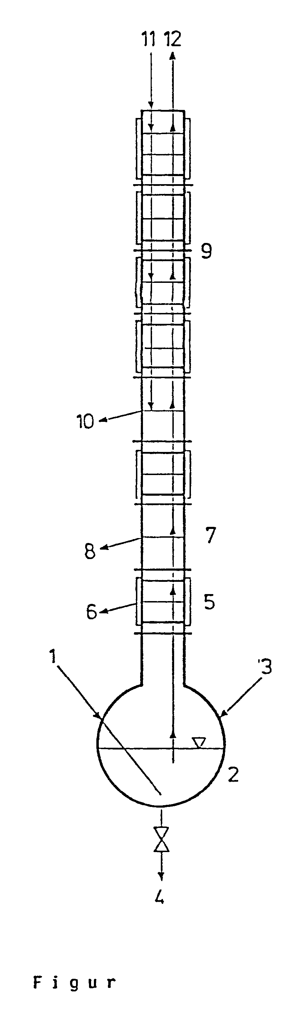

**Ansprüche**

1. Verfahren zur kontinuierlichen Abscheidung von Maleinsäureanhydrid aus gasförmigen Reaktionsgemischen, die man bei der katalytischen Oxidation von Kohlenwasserstoffen erhält, durch Behandlung der gasförmigen Reaktionsgemische mit Lösungsmitteln, dadurch gekennzeichnet, daß man das das Maleinsäureanhydrid enthaltende gasförmige Reaktionsgemisch mit einem einwertigen aliphatischen Alkohol mit 1 bis 3 oder 5 bis 8 Kohlenstoffatomen in Kontakt bringt, die dabei anfallenden gasförmigen Stoffe im Gegenstrom mit einem Dicarbonsäurediester in Berührung bringt und das flüssige Verfahrensprodukt dem Sumpf entnimmt.

2. Verfahren nach Anspruch 1, dadurch gekennzeichnet, daß man, bezogen auf 1 Mol Maleinsäureanhydrid, 0,2 bis 10 Mol Alkohol und 0,2 bis 20 Mol Dicarbonsäurediester anwendet.

3. Verfahren nach Anspruch 1, dadurch gekennzeichnet, daß man das das Maleinsäureanhydrid enthaltende gasförmige Reaktionsgemisch und den Alkohol in den unteren Teil einer Kolonne einleitet, Wasser oberhalb dieser Einleitung aus der Kolonne ausschleust, den Dicarbonsäurediester über den Kopf der Kolonne zuführt und das flüssige Verfahrensprodukt dem Kolonnensumpf entnimmt.

4. Verfahren nach Anspruch 1, dadurch gekennzeichnet, daß man das das Maleinsäureanhydrid enthaltende gasförmige Reaktionsgemisch und den Alkohol in den unteren Teil einer Kolonne einleitet, aus dem mittleren Teil der Kolonne flüssiges Gemisch ausschleust, aus diesem Gemisch leichter als der Dicarbonsäurediester siedende Anteile abdestilliert und in den unteren Teil der Kolonne einleitet, und den bei dieser Destillation als Sumpfprodukt anfallenden Dicarbonsäurediester über den Kopf der Kolonne zuführt.

5. Verfahren nach Anspruch 1, dadurch gekennzeichnet, daß man als Dicarbonsäurediester einen Diester von C$_4$-Dicarbonsäuren verwendet.

6. Verfahren nach Anspruch 1, dadurch gekennzeichnet, daß man als Dicarbonsäurediester einen Ester aus der Bernstein-, Fumar- oder Maleinsäure und dem eingesetzten Alkohol verwendet.

7. Verfahren nach Anspurch 1, dadurch gekennzeichnet, daß man als Alkohol Methanol, Ethanol, Propanol, Isopropanol, n-Amylalkohol, iso-Amylalkohol, n-Hexanol oder 2-Ethylhexanol verwendet.

8. Verfahren nach Anspruch 1, dadurch gekennzeichnet, daß man als Alkohol n- oder iso-Amylalkohol verwendet.

**Claims**

1. A process for continuously removing maleic anhydride from gaseous reaction mixtures obtained on catalytic oxidation of hydrocarbons, by treating the gaseous reaction mixtures with solvents, which comprises bringing the gaseous reaction mixture containing the maleic anhydride into contact with a monohydric aliphatic alcohol having from 1 to 3 or from 5 to 8 carbon atoms, bringing the gaseous substances produced into contact, in countercurrent, with a dicarboxylic acid diester, and removing the liquid product from the bottom of the column.

2. A process as claimed in claim 1, wherein from 0.2 to 10 mol of alcohol and from 0.2 to 20 mol of dicarboxylic acid diester are used, based on 1 mol of maleic anhydride.

3. A process as claimed in claim 1, wherein the alcohol and the gaseous reaction mixture containing the maleic anhydride are introduced into the lower part of a column, water is withdrawn from the column above this point of introduction, the dicarboxylic acid diester is fed in at the head of the column, and the liquid product is removed from the bottom of the column.

4. A process as claimed in claim 1, wherein the alcohol and the gaseous reaction mixture containing the maleic anhydride are introduced into the lower part of a column, a liquid mixture is withdrawn from the central part of the column, the components of this mixture which have a lower boiling point than the dicarboxylic acid diester are removed by distillation and introduced into the lower part of the column, and the dicarboxylic acid diester produced as a bottom product in this distillation is fed in at the head of the column.

5. A process as claimed in claim 1, wherein the dicarboxylic acid diester used is a diester of a C$_4$-dicarboxylic acid.

6. A process as claimed in claim 1, wherein the dicarboxylic acid diester used is an ester formed from succinic acid, fumaric acid or maleic acid and the alcohol employed.

7. A process as claimed in claim 1, wherein the alcohol used is methanol, ethanol, propanol, isopropanol, n-amyl alcohol, isoamyl alcohol, n-hexanol or 2-ethylhexanol.

8. A process as claimed in claim 1, wherein the alcohol used is n- or isoamyl alcohol.

**Revendications**

1. Procédé de séparation continue de l'anhydride maléique de mélanges réactionnels gazeux, que l'on obtient au cours de l'oxydation catalytique d'hydrocarbures, par le traitement des mélanges réactionnels gazeux par des solvants, caractérisé en ce que l'on met le mélange réactionnel gazeux contenant l'anhydride maléique en contact avec un alcool aliphatique monohydroxylé, qui comporte 3 ou 5 à 8 atomes de carbone, on amène les substances gazeuses présentes de ce fait en contact, en contrecourant, avec un diester d'un acide dicarboxylique et on soutire le produit liquide résultant par le fond.

2. Procédé suivant la revendication 1, caractérisé en ce que l'on utilise de 0,2 à 10 moles d'alcool et de 0,2 à 20 moles de diester de l'acide dicarboxylique par mole d'anhydride maléique.

3. Procédé suivant la revendication 1, caractérisé en ce que l'on introduit le mélange réactionnel gazeux contenant l'anhydride maléique et l'alcool par la partie inférieure d'une colonne, on évacue l'eau de la colonne au-dessus de l'endroit d'introduction précité, on amène le diester de l'acide dicarboxylique par la tête de la colonne et on soutire le produit liquide résultant par le fond de la colonne.

4. Procéde suivant le revendication 1, caractérisé en ce que l'on introduit le mélange réactionnel gazeux contenant l'anhydride maléique et l'alcool par la partie inférieure d'une colonne, on évacue un mélange liquide par la partie médiane de la colonne, on sépare de ce mélange les fractions à point d'ébullition inférieur à celui du diester de l'acide dicarboxylique par distillation et on les introduit dans la partie inférieure de la colonne et on amène le diester de l'acide dicarboxylique obtenu à titre de résidu au cours ce cette distillation par la tête de la colonne.

5. Procédé suivant la revendication 1, caractérisé en ce que l'on utilise un diester d'acides dicarboxyliques en $C_4$ à titre de diester de l'acide dicarboxylique.

6. Procédé suivant la revendication 1, caractériré en ce que l'on utilise un ester de l'acide succinique, de l'acide fumarique ou de l'acide maléique et de l'alcool mis en oeuvre à titre de diester de l'acide dicarboxylique.

7. Procédé suivant la revendication 1, caractérisé en ce que l'on utilise le méthanol, l'éthanol, le propanol, l'isopropanol, l'alcool n-amylique, l'alcool isoamylique, le n-hexanol ou le 2-éthylhexanol, à titre d'alcool.

8. Procédé suivant la revendication 1, caractérisé en ce que l'on utilise l'alcool n-amylique ou l'alcool isoamylique a titre d'alcool.

11 12

9

10

8 7

6 5

1 3

2

4

F i g u r